# EUROPÄISCHE PATENTSCHRIFT

(11) **EP 3 320 078 B1**
(45) Veröffentlichungstag und Bekanntmachung des Hinweises auf die Patenterteilung: **29.04.2020**
(21) Anmeldenummer: 16721078.0
(22) Anmeldetag: 27.04.2016
(51) Int. Cl.: G01D 3/10, G01N 35/00, C12M 1/00, C12M 1/36

(54) **VERFAHREN ZUM MESSEN EINER MEHRZAHL VON ZUSTANDSPARAMETERN EINES IN EINEM BEHÄLTER ENTHALTENEN FLUIDS**
MEASURING METHOD OF A PLURALITY OF PARAMETERS OF A FLUID IN A CONTAINER
METHODE DE MESURE D'UNE PLURALITE DE PARAMETRES D'UN FLUDIE CONTENU DANS UN RECIPIENT

(30) Priorität: 06.07.2015 DE 102015110893
(43) Veröffentlichungstag der Anmeldung: 16.05.2018
(73) Patentinhaber: Sartorius Stedim Biotech GmbH, 37079 Göttingen (DE)
(72) Erfinder: BECKER, Mario, 37085 Göttingen (DE); GRELLER, Gerhard, 37085 Göttingen (DE); GRIMM, Christian, 37308 Heilbad Heiligenstadt (DE); ADAMS, Thorsten, 37085 Göttingen (DE); BÖTTCHER, Lars, 34212 Melsungen (DE); WEICHERT, Henry, 04720 Westewitz (DE)
(74) Vertreter: Schneider, Peter Christian
(86) Internationale Anmeldenummer: PCT/EP2016/000679
(87) Internationale Veröffentlichungsnummer: WO 2017/005333

(56) Entgegenhaltungen:
- EP-A1- 2 365 575
- EP-B1- 2 503 320
- DE-B3-102006 054 165
- US-A1- 2004 030 417
- US-A1- 2013 084 030

## Beschreibung

### Gebiet der Erfindung

Die Erfindung betrifft ein Verfahren zum Messen einer Mehrzahl von Zustandsparametern eines in einem Behälter enthaltenen Fluids, wobei der zum Einmalgebrauch konfigurierte Behälter eine Wandung aufweist, an der eine Sensorträgerplatte fluiddicht festgelegt ist, welche eine Mehrzahl von Sensoren trägt, die einerseits mit dem Innenraum des Behälters in Wirkkontakt und andererseits mit einer externen Steuereinheit, die Messdaten von den Sensoren empfängt und verarbeitet, in datenaustauschender Verbindung stehen.

### Stand der Technik

Ein derartiges Verfahren ist bekannt aus der EP 2 503 320 B1 sowie aus der US 2013/0084030 A1.

Die oben erstgenannte Druckschrift offenbart einen Bioreaktor in Form eines Einweg-Bioreaktorbeutels, d.h. eines zum Einmalgebrauch als Bioreaktor konfigurierten Beutels mit flexiblen Wänden. Derartige Einweg-Bioreaktorbeutel finden zunehmend Eingang in die Produktionsprozesse der biochemischen und pharmazeutischen Industrie. Gegenüber den früher üblichen, starren und mehrfach verwendbaren Behältnissen haben die auch als Single-use-bags bezeichneten Einwegbeutel erhebliche Vorteile in Hinsicht auf Hygiene und Effizienz sowie Flexibilität von Arbeitsprozessen. Nicht unproblematisch ist dabei jedoch die Überwachung der im Beutelinneren ablaufenden Reaktionsprozesse. Dies erfolgt stets mit geeigneten Sensoren, wie bspw. elektrochemischen-Sensoren, Thermo- und Leitfähigkeitssensoren, optischen und optochemischen Sensoren etc. Für jeden speziellen Reaktionsprozess im Beutelinneren müssen spezielle Sensoren zu seiner Überwachung vorgesehen sein.

Aus der EP 2 829 598 A2 ist es bekannt, die Einweg-Bioreaktorbeutel in Anlehnung an die auch früher im Zusammenhang mit starren Behältnissen verwendete Technik mit standardisierten Ports zu versehen, die eine Schnittstelle zwischen Beutelinnerem und Beuteläußerem bieten und in die bedarfsweise geeignete Sensoren eingesteckt werden. Deren Sensorkopf ragt dann in das Beutelinnere hinein, um den für den speziellen Messvorgang erforderlichen Kontakt mit dem Fluid im Beutelinneren herzustellen. Der außerhalb des Beutels angeordnete Sensorteil bietet die Schnittstelle zu einer Kommunikationsstrecke zu einer externen Steuereinheit. Diese Kommunikationsstrecke kann bspw. als elektrisches Kabel, als Glasfaserkabel oder drahtlos, bspw. in Form einer Funk- oder IR-Strecke, ausgebildet sein. Über diese Kommunikationsstrecke stehen Sensor und externe Steuereinheit in datenaustauschender Verbindung, sodass vom Sensorkopf erzeugte Daten analoger oder digitaler Natur über die Kommunikationsstrecke an die Steuereinheit versendet und dort weiterverarbeitet werden können. Die genannte Druckschrift offenbart als mechanische Schnittstelle als sogenannten Multi-Port eine stoffschlüssig in die Beutelwandung eingebrachte Platte mit einer Mehrzahl von Einzel-Durchbrüchen, in die die für den speziellen Reaktionsprozess benötigten Sensoren eingesteckt werden. Der Schritt des Einsteckens eines Sensors in einen der Ports ist jedoch unter hygienischen Aspekten, z.B. unter Aspekten der Wahrung der Sterilität des Beutelinneren, kritisch. Hier kann die Gefahr bestehen, dass bei unsachgemäßem Vorgehen Verschmutzungen in das Beutelinnere eindringen und das Fluid kontaminieren können.

Eine im Hinblick auf die Flexibilität der realisierbaren Sensorkonfigurationen noch konsequenter ausgestaltete Vorrichtung ist aus der US 2013/ 0084030 A1 bekannt. Dort werden nicht nur austauschbare Sensoren, sondern sogar austauschbare Ports zum Einstecken unterschiedlicher Sensoren offenbart. Die oben erläuterte Hygiene-Problematik bleibt hierbei jedoch unverändert bestehen.

Mit der Entwicklung kostengünstiger und daher für den Einmalgebrauch geeigneter Sensoren ist es möglich geworden, die fraglichen Einwegbehälter, die als Beutel mit flexibler Wandung oder als sogenannte "Carboy"-Behälter aus Kunststoff mit starrer Wandung erhältlich sind, bereits herstellerseitig mit Einmal-Sensoren auszustatten, die unlösbar auf einer fluiddicht - z.B. durch stoffschlüssige Verbindung, etwa Verkleben oder Verschweißen, oder aber auch durch geeignete kraft- oder formschlüssige Verbindung - mit der Wandung verbundenen Sensorträgerplatte angeordnet sind. Die Konfektionierung, d.h. die Ausstattung eines individuellen Einwegbehälters mit einer individuellen Zusammenstellung unterschiedlicher Sensoren kann dann unter hygienisch optimierten Bedingungen und unter Wahrung der Sterilität des Behälterinneren auf Seiten des Beutelherstellers nach Standard-Vorgaben oder speziell nach Kundenwunsch erfolgen. Entsprechende Einweg-Bioreaktorbeutel sind aus der eingangs genannten, gattungsbildenden EP 2 503 320 B1 bekannt. Problematisch ist jedoch die fehlende Austauschbarkeit der Sensoren, die im Fall eines Sensor-Defektes zum Verwerfen des gesamten Beutels samt seines teuren Inhalts führen kann. Dies ist umso ärgerlicher, wenn sich nachträglich herausstellen sollte, dass gar kein Sensordefekt, sondern nur eine korrigierbare Abweichung im Reaktionsprozess vorlag, die zu einen Sensordefekt nahelegenden Messwerten geführt hatte.

Aus der DE 2006 054 165 B3 sind Wasserstoffsensoren bekannt, die zur Verbesserung von Robustheit und Langlebigkeit nicht nur ein eigentliches, optisches Messsignal, sondern zugleich Korrektursignale anderen Ursprungs aufnehmen und zur Korrektur bzw. Kalibrierung des eigentlichen Messsignals einsetzen.

Aus der US 2004/0030417 A1 ist das Prinzip der sog. passiven Redundanz bekannt, bei dem für einen zu messenden Parameter mehrere baugleiche Sensoren vorgehalten aber zu jedem Zeitpunkt nur einer aktiv ist. Wird mittels eines komplexen, modellbasierten Fehlerabschätzungssystems ein Defekt des aktiven Sensors festgestellt, wird er deaktiviert und sein Duplikat kommt fortan zum Einsatz.

### Aufgabenstellung

Es ist die Aufgabe der vorliegenden Erfindung, ein Verfahren zur Verfügung zu stellen, welches die durch tatsächliche oder scheinbare Sensordefekte verursachten Verluste reduziert und insbesondere in der Lage ist, die eine von der anderen Situation zu unterscheiden.

### Darlegung der Erfindung

Diese Aufgabe wird in Verbindung mit den Merkmalen des Oberbegriffs von Anspruch 1 dadurch gelöst, dass die Sensorplatte von wenigstens einem der Sensoren ein temporär inaktives Duplikat trägt, welches dann aktiviert wird, wenn Messdaten des Sensors im Rahmen eines von einer externen Steuereinheit durchgeführten Integritäts- oder Plausibilitätstests als auffällig eingestuft werden, wobei die Steuereinheit temporär sowohl die von dem Sensor als auch die von dem Duplikat empfangenen Messdaten verarbeitet und wobei im Fall, dass die Verarbeitung der einerseits von dem Sensor und andererseits von dem Duplikat empfangenen Messdaten zu innerhalb einer vorgegebenen Toleranz gleichen Ergebnissen führt, das Duplikat wieder deaktiviert wird und im Fall, dass die Verarbeitung der einerseits von dem Sensor und andererseits von dem Duplikat empfangenen Messdaten zu unter Berücksichtigung der vorgegebenen Toleranz unterschiedlichen Ergebnissen führt, der Sensor deaktiviert wird.

Bevorzugte Ausführungsformen sind Gegenstand der abhängigen Ansprüche.

Die kostengünstige Produktionsweise moderner Einweg-Sensoren erlaubt die mehrfache Ausführung einzelner Sensoren eines größeren Sensorfeldes, ohne dass sich der Gesamtpreis eines entsprechenden Einweg-Behälters, z. B. eines Einweg-Bioreaktors oder Einweg-Mischbehälters in Form eines Beutels oder starren "Carboy"-Behälters, dadurch wesentlich erhöhen würde. Erfindungsgemäß ist daher von wenigstens einem der Sensoren des Sensorfeldes, bevorzugt von mehreren Sensoren des Sensorfeldes, besonders bevorzugt von allen Sensoren des Sensorfeldes jeweils ein Duplikat vorgesehen, welches für die bestimmungsgemäße Messung von Zustandsparametern des Fluides im Behälter, z.B. einem Einweg-Bioreaktorbeutel oder einem Einweg-Mischbehälter, nicht zwingend erforderlich ist. Die Duplikate dienen vielmehr lediglich als Reserve-Sensoren, die im Fall eines Defektes des jeweiligen Primär-Sensors zum Einsatz kommen können, ohne dass dabei der Produktionsprozess unterbrochen oder durch einen aufwendigen Sensorwechsel gefährdet werden müsste. Es wird also gezielt ein mechanischer und elektronischer "Überbau" im Sinne eines Überschusses an Sensoren angeboten, dessen Mehrkosten jedoch durch den Gewinn an Produktionssicherheit deutlich überkompensiert werden.

Wie oben bereits erläutert, ist es nicht immer einfach, einen tatsächlichen Sensordefekt von einer temporären und korrigierbaren Entgleisung des Produktionsprozesses zu unterscheiden. Erfindungsgemäß wird daher eine Strategie angeboten, wie die bereitgestellten Reserve-Sensoren sinnvoll eingesetzt werden können. Voraussetzung für jede Art ihres sinnvollen Einsatzes ist, dass Ausnahmesituationen, die durch Sensordefekt oder durch Prozessentgleisung verursacht sein können, überhaupt detektiert werden. Erfindungsgemäß ist daher zunächst vorgesehen, dass die externe Steuereinheit die mittels der Primär-Sensoren erfassten Messdaten kontinuierlich, periodisch oder sporadisch Integritätstests oder Plausibilitätstests unterwirft. Ein derartiger Integritäts- bzw. Plausibilitätstest kann beispielsweise im Vergleich aktueller Messdaten mit gespeicherten oder aus anderen Messdaten errechneten Referenzdaten bestehen. Im einfachsten Fall wird eine Liste von zu gegebenen Zeitpunkten zu erreichenden Referenzwerten hinterlegt, die dann zu den entsprechenden Zeitpunkten mit den aktuellen Messdaten verglichen werden. In einem komplizierteren Fall ist es jedoch auch möglich, aus Messdaten eines oder mehrerer erster Sensoren Rückschlüsse auf bestimmte Zusatzparameter des Fluides, die per se einer Messung nur schwer oder gar nicht zugänglich sind, im Einweg-Behälter zu ziehen und die prognostizierten Parameterwerte mit tatsächlichen Messwerten entsprechender zweiter Sensoren zu vergleichen. Weiter ist es auch möglich, aktuelle Messdaten aus einem parallel ablaufenden Referenzprozess als Referenzwerte oder als Basis zur Berechnung von Referenzwerten heranzuziehen. Die konkrete Ausgestaltung der Integritäts- bzw. Plausibilitätstests muss jeweils von der konkreten Gestaltung des Reaktionsprozesses abhängen. Ergebnis eines solchen Integritätstests ist in jedem Fall die Entscheidung, ob eine Ausnahmesituation vorliegt oder nicht, wobei das Vorliegen einer Ausnahmesituation dann die nachfolgend erläuterte Korrekturstrategie auslöst.

So ist erfindungsgemäß vorgesehen, dass die Steuereinheit temporär sowohl die von dem Sensor als auch die von dem Duplikat empfangenen Messdaten verarbeitet. Mit anderen Worten werden der Primärsensor und sein Duplikat für eine gewisse Zeit parallel betrieben. Für den Fall, dass die Verarbeitung der einerseits von dem Sensor und andererseits von dem Duplikat empfangenen Messdaten zu innerhalb einer vorgegebenen Toleranz gleichen Ergebnissen führt, ist vorgesehen, dass das Duplikat wieder deaktiviert wird. Liefern nämlich Sensor und Duplikat im Wesentlichen die gleichen Messdaten, kann davon ausgegangen werden, dass die durch den Integritätstest erkannte Ausnahmesituation nicht das Resultat eines Sensordefektes ist. Die Wahrscheinlichkeit, dass sowohl Sensor als auch Duplikat defekt sind und zusätzlich in derselben Weise defekt sind, sodass beide gleiche, falsche Messdaten liefern, kann als extrem gering eingestuft werden. Erfindungsgemäß wird in einem solchen Fall der durch Prozessentgleisung verursachten Ausnahmesituation das Duplikat und nicht den PrimärSensor wieder deaktiviert. Dies wird insbesondere vor dem Hintergrund einer maximalen Datenkonsistenz als vorteilhaft angesehen. Der Fachmann wird verstehen, dass in diesem Fall zur Behebung der Ausnahmesituation prozesstechnische Maßnahmen erforderlich sind, die die Prozessentgleisung, welche zu der erkannten Ausnahmesituation geführt hat, korrigieren.

Andererseits ist vorgesehen, dass im Fall, dass die Verarbeitung der einerseits von dem Sensor und andererseits von dem Duplikat empfangenen Messdaten zu unter Berücksichtigung der vorgegebenen Toleranz unterschiedlichen Ergebnissen führt, der Sensor deaktiviert wird. Führt also, mit anderen Worten, der Sensorvergleich zu dem Ergebnis, dass die durch den Plausibilitätstest erkannte Ausnahmesituation auf einen Defekt des Primär-Sensors zurückzuführen ist, wird der defekte Sensor abgeschaltet und der Prozess unter Verwendung des Duplikats fortgesetzt. In diesem Fall besteht keine Notwendigkeit für prozesstechnische Maßnahmen.

Bei einer günstigen Weiterbildung der Erfindung ist vorgesehen, mehrere Sensoren des Sensorfeldes gemeinsam zur Realisierung eines sogenannten "Soft-Sensors" zu nutzen. Hierbei ist vorgesehen, dass für einen von keinem der Sensoren direkt messbaren Zustandsparameter ein Parameterwert von der externen Steuereinheit aus Messdaten mehrerer unterschiedlicher Sensoren berechnet wird. So sind wichtige Zustandsparameter, wie beispielsweise der Biomasseanteil im Fluid, denkbar, für die keine unmittelbare Sensorik existiert. In Kenntnis verschiedener Prozessparameter und der Messdaten mehrerer Sensoren, beispielsweise eines O₂-Sensors, eines CO₂-Sensors, eines Sensors für die optische Dichte des Fluids etc., lässt sich jedoch der gesuchte Parameter rechnerisch bestimmen. Mehrere Einzelsensoren wirken daher als komplexer "Soft-Sensor" für den komplexen Zustandsparameter zusammen.

Bei dem für das erfindungsgemäße Verfahren verwendeten Behälter kann es sich vorzugsweise um einen Einweg-Bioreaktor oder um einen Einweg-Mischbehälter handeln, die jeweils als Beutel mit wenigstens bereichsweise flexibler Wandung oder als Kunststoffbehälter ("Carboy") mit wenigstens bereichsweiser starrer Wandung ausgelegt sein können.

Weitere Merkmale und Vorteile der Erfindung ergeben sich aus der nachfolgenden speziellen Beschreibung und den Zeichnungen.

### Kurzbeschreibuna der Zeichnungen

Es zeigt:

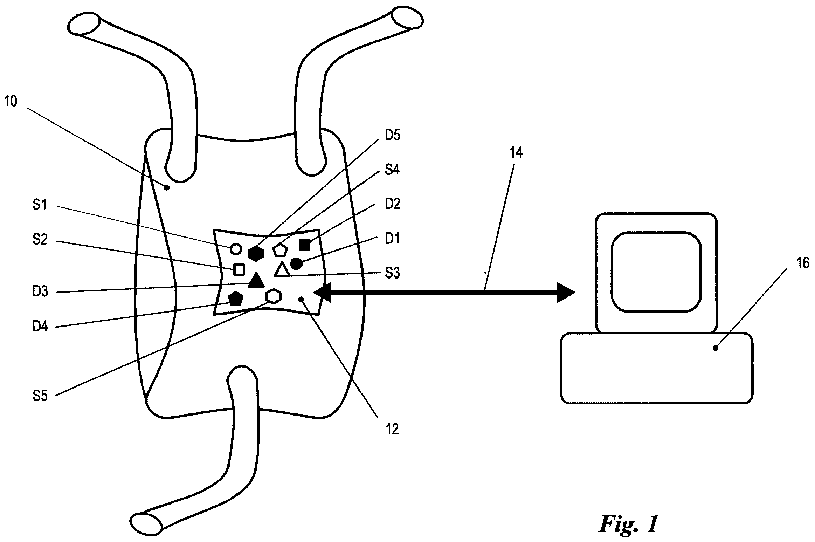

- Figur 1:: Figur 1 eine schematische Darstellung eines an eine externe Steuereinheit angeschlossenen Behälters.

### Ausführliche Beschreibung bevorzugter Ausführungsformen

Figur 1 zeigt in stark schematisierter Darstellung einen Behälter, z.B. einen Einweg-Bioreaktorbeutel oder einen Einweg-Mischbehälter 10, der ein Sensorfeld 12 trägt. Das Sensorfeld 12 umfasst eine im Detail nicht näher dargestellte, mit der Wandung des Behälters 10 fluiddicht verbundene Trägerplatte, die vorzugsweise mit der Beutelwandung verschweißt ist. Bei anderen Ausführungsformen der Erfindung kann die Verbindung auch kraft- oder formschlüssig ausgebildet sein; relevant ist lediglich ihre fluiddichte Eigenschaft. Auf der Trägerplatte ist eine Mehrzahl von Sensoren S1-S5 angeordnet, die mit einem nicht näher dargestellten Sensorkopf in Wirkkontakt mit dem Beutelinneren, insbesondere mit einem im Beutelinneren befindlichen Fluid, stehen. Jeder Sensor S1-S5 ist zudem über eine allgemein mit dem Kommunikationspfeil 14 angedeutete Kommunikationsstrecke mit einer externen Steuereinheit 16 verbunden. Über die Kommunikationsstrecke 14 können Daten und Steuerbefehle zwischen der externen Steuereinheit 16 und den Sensoren S1-S5 ausgetauscht werden. Die Kommunikationsstrecke 14 kann kabelgebunden sein, beispielsweise als elektrisches Kabel und/oder optisches Faserkabel ausgeführt. Alternativ oder zusätzlich kann wenigstens einer der Sensoren S1-S5 drahtlos, insbesondere über eine Funk- oder IR-Strecke, mit der externen Steuereinheit 16 verbunden sein. Die Sensoren S1-S5 dienen unterschiedlichen Messaufgaben, deren spezielle Art für die vorliegende Erfindung nicht von Belang ist. Deren Unterschiedlichkeit in Figur 1 jedoch durch die unterschiedliche Formgebung der Sensoren S1-S5 angedeutet ist.

Weiter umfasst das Sensorfeld 12 zu den Sensoren S1-S5 baugleiche Duplikate D1-D5, die jeweils einem der Sensoren S1-S5 (angedeutet durch die gleiche Formgebung in Figur 1) zugeordnet sind. Auch die Duplikate D1-D5 stehen über die Kommunikationsstrecke 14 mit der externen Steuereinheit 16 in Verbindung.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum von Variationsmöglichkeiten an die Hand gegeben.

### Bezugszeichenliste

- 10: Behälter
- 12: Sensorfeld
- S1-S5: Sensor auf 12
- D1-D5: Duplikat von S1-S5
- 14: Kommunikationsstrecke
- 16: externe Steuereinheit

## Patentansprüche

1. Verfahren zum Messen einer Mehrzahl von Zustandsparametern eines in einem Behälter (10) enthaltenen Fluids,
wobei der zum Einmalgebrauch konfigurierte Behälter (10) eine Wandung aufweist, an der eine Sensorträgerplatte fluiddicht festgelegt ist, welche eine Mehrzahl von Sensoren (S1-S5) trägt, die einerseits mit dem Innenraum des Behälters (10) in Wirkkontakt und andererseits mit einer externen Steuereinheit (16), die Messdaten von den Sensoren (S1-S5) empfängt und verarbeitet, in datenaustauschender Verbindung (14) stehen,
**dadurch gekennzeichnet,**
**dass** die Sensorplatte von wenigstens einem der Sensoren (S1-S5) ein temporär inaktives Duplikat (D1-D5) trägt, welches dann aktiviert wird, wenn Messdaten des Sensors (S1-S5) im Rahmen eines von der externen Steuereinheit (16) durchgeführten Integritäts- oder Plausibilitätstests als auffällig eingestuft werden, wobei die Steuereinheit (16) temporär sowohl die von dem Sensor (S1-S5) als auch die von dem Duplikat (D1-D5) empfangenen Messdaten verarbeitet und wobei im Fall, dass die Verarbeitung der einerseits von dem Sensor (S1-S5) und andererseits von dem Duplikat (D1-D5) empfangenen Messdaten zu innerhalb einer vorgegebenen Toleranz gleichen Ergebnissen führt, das Duplikat (D1-D5) wieder deaktiviert wird
und im Fall, dass die Verarbeitung der einerseits von dem Sensor (S1-S5) und andererseits von dem Duplikat (D1-D5) empfangenen Messdaten zu unter Berücksichtigung der vorgegebenen Toleranz unterschiedlichen Ergebnissen führt, der Sensor (S1-S5) deaktiviert wird.

2. Verfahren nach Anspruch 1,
**dadurch gekennzeichnet,**
**dass** für einen von keinem der Sensoren direkt messbaren Zustandsparameter ein Parameterwert von der externen Steuereinheit aus Messdaten mehrerer unterschiedlicher Sensoren berechnet wird.

3. Verfahren nach einem der vorangehenden Ansprüche,
**dadurch gekennzeichnet, dass** der Behälter ein Einweg-Bioreaktor oder ein Einweg-Mischbehälter ist.

4. Verfahren nach Anspruch 3,
**dadurch gekennzeichnet,**
**dass** der Einweg-Bioreaktor oder der Einweg-Mischbehälter als Beutel mit bereichsweise flexibler Wandung oder als "Carboy"-Kunststoffbehälter mit bereichsweise starrer Wandung ausgelegt ist.

## Claims

1. Method for measuring a plurality of status parameters of a fluid contained in a container (10), wherein the container configured for single use (10) has a wall on which a sensor support plate is fixed in a fluid-tight manner, said plate supporting a plurality of sensors (S1-S5) which are in operative contact with the interior of the container (10) on the one hand and in data-exchanging connection (14) with an external control unit (16) which receives and processes measurement data from the sensors (S1-S5) on the other hand,
**characterized in that**
the sensor plate carries a temporarily inactive duplicate (D1-D5) of at least one of the sensors (S1-S5) which is activated when measurement data of the sensor (S1-S5) are classified as conspicuous in the context of an integrity or plausibility test carried out by an external control unit (16), wherein the control unit (16) temporarily processes both the measurement data received from the sensor (S1-S5) and the data received from the duplicate (D1-D5),
and wherein, in the event that the processing of the measurement data received from the sensor (S1-S5) and from the duplicate (D1-D5) leads to identical results within a predetermined tolerance, the duplicate (D1-D5) is deactivated again and, in the event that the processing of the measurement data received from the sensor (S1-S5) and from the duplicate (D1-D5) leads to different results taking into account the predetermined tolerance, the sensor (S1-S5) is deactivated.

2. The method according to claim 1, **characterized in that**, for a status parameter that cannot be directly measured by any of the sensors, the external control unit calculates a parameter value from measurement data of several different sensors.

3. The method according to one of the preceding claims, **characterized in that** the container is a single-use bioreactor or a single-use mixing container.

4. The method according to claim 3, **characterized in that** the single-use bioreactor or the single-use mixing container is designed as a bag with walls that are flexible in some areas or as a "Carboy" plastic container with walls that are rigid in some areas.

## Revendications

1. Procédé pour mesurer une pluralité de paramètres d'état d'un fluide contenu dans un récipient (10),
ledit récipient (10) configuré pour être à usage unique présentant une paroi sur laquelle est fixée de manière étanche aux fluides une plaque de support de capteurs supportant une pluralité de capteurs (S1-S5) qui se trouvent, d'une part, en contact actif avec l'intérieur du récipient (10) et, d'autre part, en relation d'échange de données (14) avec une unité de commande externe (16) qui reçoit et traite les données de mesure venant des capteurs (S1-S5),
**caractérisée en ce**
**que** la plaque de capteurs d'au moins l'un des capteurs (S1-S5) supporte une réplique inactive temporaire (D1-D5) qui est alors activée lorsque les données de mesure du capteur (S1-S5) sont considérées comme anormales dans le cadre d'un test d'intégrité et de plausibilité réalisé par l'unité de commande externe (16), sachant que l'unité de commande (16) traite temporairement les données de mesure qu'elle reçoit du capteur (S1-S5) et de la réplique (D1-D5) et que, si le traitement des données de mesure reçues, d'une part, du capteur (S1-S5) et, d'autre part, de la réplique (D1-D5) donne les mêmes résultats dans les limites d'une tolérance prédéfinie, la réplique (D1-D5) est à nouveau désactivée et que, si le traitement des données de mesure reçues, d'une part, du capteur (S1-S5) et, d'autre part, de la réplique (D1-D5) donne des résultats différents compte tenu de la tolérance prédéfinie, le capteur (S1-S5) est désactivé.

2. Procédé selon la revendication 1,
**caractérisé en ce**
**qu'**une valeur paramétrique est calculée par l'unité de commande externe à partir des données de mesure de plusieurs capteurs différents pour un paramètre d'état ne pouvant être mesuré directement par aucun des capteurs.

3. Procédé selon l'une des revendications précédentes,
**caractérisé en ce que** le récipient est un bioréacteur à usage unique ou un réservoir de mélange à usage unique.

4. Procédé selon la revendication 3,
**caractérisé en ce**
**que** le bioréacteur à usage unique ou le réservoir de mélange à usage unique est conçu comme un sachet à paroi flexible par endroits ou comme une tourie en plastique à paroi rigide par endroits.
